# EUROPEAN PATENT SPECIFICATION

(11) **EP 4 151 144 B1**
(45) Date of publication and mention of the grant of the patent: **19.11.2025**
(21) Application number: 21804306.5
(22) Date of filing: 29.03.2021
(51) Int. Cl.: A61B 5/00, A61B 5/026, A61B 5/0295, A61B 5/024, A61B 5/0205

(54) **BIOMETRIC INFORMATION MEASUREMENT DEVICE AND BIOMETRIC INFORMATION MEASUREMENT SYSTEM**
VORRICHTUNG ZUR MESSUNG BIOMETRISCHER INFORMATIONEN UND SYSTEM ZUR MESSUNG BIOMETRISCHER INFORMATIONEN
DISPOSITIF DE MESURE D'INFORMATIONS BIOMÉTRIQUES ET SYSTÈME DE MESURE D'INFORMATIONS BIOMÉTRIQUES

(30) Priority: 11.05.2020 JP 2020083222
(43) Date of publication of application: 22.03.2023
(73) Proprietor: Sony Group Corporation, Tokyo 108-0075 (JP)
(72) Inventor: ITO Atsushi, Tokyo 108-0075 (JP); IKUTA Tomoya, Tokyo 108-0075 (JP)
(74) Representative: D Young & Co LLP
(86) International application number: PCT/JP2021/013304
(87) International publication number: WO 2021/229938

(56) References cited:
- WO-A1-2014/007210
- WO-A1-2019/133926
- WO-A1-2019/198991
- JP-A- 2010 051 790
- JP-A- 2013 009 709
- JP-A- 2017 051 340
- US-A1- 2017 000 350
- US-A1- 2017 041 564
- US-A1- 2017 319 084
- US-A1- 2021 007 617

## Description

### TECHNICAL FIELD

The present technology relates to a biological information measurement device and a biological information measurement system.

### BACKGROUND ART

A photoplethysmography (PPG) sensor, a laser Doppler flowmeter (LDF), and the like are known as a technology of measuring a pulse and blood flow rate of a human body by irradiating the human body with light emitted from a light source once and observing backscattered light. Further, those technologies are already mounted on wearable devices and the like and are widely used in the market. Biological information such as a plethysmogram and a blood flow rate reflects a change in a cardiovascular system of a human body and thus is extremely effective biological information for observing a tension state of the human body.

Further, values output by those sensors are affected by a heart rate, blood pressure, respiration, and the like of a person and thus are used for the purpose of sports and health care. However, those numerical values are affected not only by the above indices but also by a tension state of the person. Therefore, for example, Patent Document 1 discloses a technology of restraining variations of measurement results caused by various factors such as body motion, tension, stress, and a low temperature environment by installing a plurality of light receivers.

By the way, in Prior Document 1 described above, it is expected to exclude a factor caused by tension. Meanwhile, there is also a demand to measure a tension state of a person. In this case, for example, there is a technology of measuring a tension state by installing a large number of light sources. However, the technology uses a phenomenon in which a minute disturbance of a blood flow in a peripheral site occurs in each local site due to stress and thus is required to measure a state of a local blood flow. Therefore, a light receiver for performing observation tends to be inevitably small.

Further, as another disclosed technology, Prior Document 2 discloses a technology of increasing an area of a light receiver according to a distance from a light source in order to compensate for light attenuated according to the distance.

### CITATION LIST

### PATENT DOCUMENT

Patent Document 1: Japanese Patent Application Laid-Open No. 2018-68556
Patent Document 2: Japanese Patent Application Laid-Open No. 2017-51340
Other prior art is provided in JP 2013-009709A, US 2017/319084A, WO 2019/133926A and WO 2019/198991A.

### PROBLEMS TO BE SOLVED BY THE INVENTION

In a case where a human body is measured by using a sensor, it is desired to measure not only disturbance of a local blood flow, but also conventional indices such as a heart rate, a blood pressure, and respiration at the same time in many cases. Further, it is known that the indices such as the heart rate are also affected by tension and stress, and thus it is expected that a tension state can be grasped with higher accuracy by using not only information regarding the disturbance of the local blood flow but also information regarding the blood pressure.

However, in a case where information regarding the heart rate, the blood pressure, and the like is measured, the disturbance of the local blood flow becomes a factor of noise. Thus, it is desirable to perform measurement in a wider range to eliminate an influence of the disturbance of the local blood flow. Further, an excessively small light receiver tends to be affected by electrical noise. Therefore, in general, in a case where those sensors are used as a sensor for measuring the heart rate, a light receiver having a relatively large area is used in many cases. Further, in order to simply create a sensor that satisfies both of those ideas, it is conceivable to add signals from a large number of small light receivers and operate the light receivers as a single large light receiver. However, in order to obtain an area equivalent to a large area of a light receiver by using light receivers having small areas as described above, it is necessary to install an extremely large number of light receivers, which is not realistic.

Further, in a case where the area of the light receiver is increased according to the distance from the light source, signals observed by those light receivers are essentially different from each other because the technology itself intends to obtain signals from subcutaneous blood vessels having different depths. For example, in a case where signals by two light receivers are different, it is difficult to separate whether a mental blood flow disturbance is observed or a difference in response in a depth direction to some stimulus is observed.

In view of the above circumstances, a main object of the present technology is to provide a technology capable of accurately measuring biological information with a simple structure.

### SOLUTIONS TO PROBLEMS - SUMMARY OF THE INVENTION

First, the present technology provides a biological information measurement device according to claim 1.

Further embodiments are as defined by the dependent claims.

### BRIEF DESCRIPTION OF DRAWINGS

Fig. 1 is a graph schematically showing a phenomenon in which, in a case where no stress is loaded, almost all blood vessels move in a uniform manner, and thus light receivers have a high correlation, whereas, when stress is loaded, physiological indices such as a blood flow rate change according to constriction force of each blood vessel, and thus the correlation between the light receivers decreases.
Fig. 2 is a schematic diagram of an arrangement example 1.
Fig. 3 is a schematic diagram of an arrangement example 2.
Fig. 4 is a block diagram of a configuration example of a processing unit 100.

### MODE FOR CARRYING OUT THE TECHNOLOGY

Hereinafter, preferred modes for carrying out the present technology will be described with reference to the drawings. Embodiments described below show examples of representative embodiments of the present technology, and the scope of the present technology is not limitedly interpreted by the embodiments. Note that description will be provided in the following order.
1. Biological information measurement device 10 according to present technology
   1-1. Configuration of biological information measurement device 10
   1-2. Arrangement example 1
   1-3. Arrangement example 2
   1-4. Processing unit 100
2. Biological information measurement system according to present technology

### 1. Biological information measurement device 10 according to present technology

Hereinafter, a configuration of a biological information measurement device 10 according to the present embodiment will be described. Note that the embodiment shows a suitable example of the present technology, but the biological information measurement device 10 according to the present technology is not limited to this configuration.

### 1-1. Configuration of biological information measurement device 10

The biological information measurement device 10 according to the present embodiment includes a light emitting element 11 that irradiates a living body with light and a plurality of light receiving elements 12 that receives light scattered in the living body, and at least one light receiving element 12 has a different area from that of another light receiving element 12. Further, the biological information measurement device may include a processing unit 100 including an analysis unit 103 and a control unit 104 as necessary.

In the present technology, because the plurality of light receiving elements 12 has different areas as described above, it is possible to calculate biological information with high accuracy even with a simple structure.

The biological information measurement device 10 according to the present embodiment is worn on, for example, a part of skin of the living body (e.g. human body) and may have various forms such as, for example, a wristband type, earring type, ring type, necklace type, attachment type, and supporter type.

In the present specification, the word "biological information" means various kinds of information regarding the living body. The living body includes, for example, a human body and also includes bodies of animals and the like other than a human. In the present technology, the biological information can be preferably a state of the autonomic nerve serving as an index of stress or tension.

The light emitting element 11 irradiates the living body with light. In the present embodiment, at least one light emitting element 11 is provided.

Examples of the light emitting element include laser light sources such as a light emitting diode (LED), an edge emitting laser (LD), and a surface emitting laser (VCSEL).

Further, for example, in a case where the light emitting element is a laser light source that emits coherent light, the light emitting element can be used for measuring biological information such as a plethysmogram and a blood flow rate. Further, for example, in a case where the light emitting element is an LED that emits non-coherent light, the light emitting element can be used for measuring biological information such as a plethysmogram. In the present technology, light emitted from the light emitting element may be visible light or non-visible light (e.g. infrared light).

It is possible to use, for example, a wavelength in a visible region, a near-infrared region, or an infrared region as a wavelength of the light emitting element.

The light receiving elements 12 receive light scattered at a plurality of measurement sites in the living body. In the present embodiment, at least two light receiving elements 12 are provided.

The light receiving element includes, for example, a photodiode (PD), a phototransistor, and the like.

Specifically, as the light receiving element, it is possible to adopt, for example, a multi-divided PD having a plurality of light receiving regions arranged one-dimensionally or two-dimensionally, a line sensor in which pixels including a PD are one-dimensionally arranged, an image sensor in which pixels including a PD are two-dimensionally arranged, or the like.

Note that, in general, in a case of a photoplethysmography (PPG) sensor that detects a heart rate, an LED is used as the light emitting element, and a PD is used as the light receiving element. A blood flow flowing through a human body pulsates, and thus a density of red blood cells under the skin is partially high and partially low. Therefore, when light having a wavelength absorbed by red blood cells is emitted and an amount of returned light is observed, the amount of light slightly changes. A heart rate monitor calculates the heart rate by using the fact that the change in the amount of light is equal to a beat of a heart.

Further, in general, in a case of a laser Doppler flowmeter (LDF), an LD is used as the light emitting element, and a PD is used as the light receiving element. When the skin is irradiated with light, part of the light is scattered by red blood cells in blood under the skin. At this time, when the red blood cells are moving, the scattered light causes the Doppler shift, and a wavelength thereof slightly changes. Meanwhile, light reflected by a non-moving tissue does not cause the Doppler shift. The light is laser light and thus has high coherence, and, as a result, a beat signal corresponding to a wavelength difference of the Doppler shift is generated in a light receiver. An LDF sensor calculates the blood flow rate by analyzing the beat signal.

In the present embodiment, the plurality of light receiving elements 12 includes a first light receiving element 121 and a second light receiving element 122.

Here, in general, many sympathetic nerves are distributed in arterioles, and it is considered that, when the sympathetic nerves are subjected to stress stimulation, norepinephrine (NE) that is a neurotransmitter is released from nerve terminals and acts on α1 receptors of vascular smooth muscle cells to cause vasoconstriction. Meanwhile, it is known that smooth muscle cells in the arterioles are sparsely distributed and that the arterioles lead to capillaries while a density of the smooth muscle cells gradually decreases toward a periphery.

Therefore, it is considered that constriction of the arterioles caused by stress depends on the density of the smooth muscle cells of each blood vessel, and thus each blood vessel shows different responses. That is, when stress is loaded, the arterioles in which many smooth muscles are distributed strongly constrict, and an amount of constriction decreases in a blood vessel near capillaries, i.e., a blood vessel in which smooth muscle is sparse. By using this fact to observe each blood vessel, it is possible to sense a stress response of a human body. For example, there is a phenomenon in which, in a case where no stress is loaded, almost all blood vessels move in a uniform manner, and thus the light receivers have a high correlation, whereas, when stress is loaded, physiological indices such as a blood flow rate change according to constriction force of each blood vessel, and thus the correlation between the light receivers decreases.

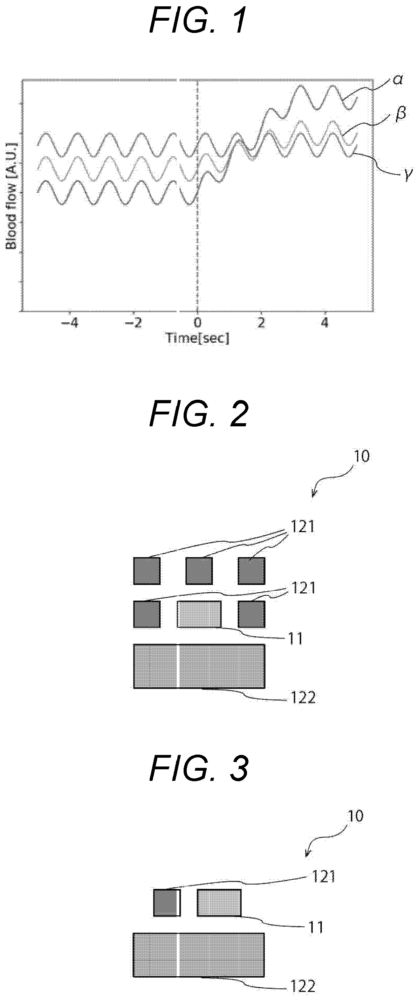

Fig. 1 is a graph schematically showing the phenomenon. With a time 0 as a boundary, a negative time is a rest time, and a positive time is a time when a stress load is applied. A vertical axis represents, for example, a blood flow rate. In this case, at the rest time, each blood vessel pulsates at a blood flow rate specific to the blood vessel (which is determined by, for example, an inner diameter or the like). When stress is applied, a blood vessel represented by γ is less likely to constrict because the amount of smooth muscle is small. Thus, the blood flow rate does not greatly change. Meanwhile, a line represented by α with a high density of smooth muscles indicates a blood vessel in which there are many smooth muscles, and the blood flow rate greatly increases due to the stress. As a result, for example, a correlation represented in the order of γ-β-α at the rest time is replaced with a correlation of α-β-γ when the stress is applied. Thus, it is possible to determine whether or not the stress is loaded.

By the way, as to how to distinguish each blood vessel, the arterioles generally have a diameter of 100 to 200 µm, and thus, if a light receiver larger than the diameter is used, a plurality of blood vessels is simultaneously measured. This makes it difficult to observe individual blood vessels to identify stress as described above. Therefore, an area of the light receiver is preferably as small as possible.

In the present embodiment, the first light receiving element 121 has an area of 0.1 mm² or less.

By the way, it is widely known that a reaction derived from the heart, such as an increase in heart rate or an increase in blood pressure, is also simultaneously observed when a stress load is applied. This phenomenon is widely known, and thus many wearable devices and the like provided with a PPG sensor are commercially available as a device for displaying a stress state. In this case, it is required to prolong a life of a battery, and thus a photodiode having a relatively large area is used to improve light utilization efficiency.

In the present embodiment, the second light receiving element 122 preferably has an area exceeding 1 mm², and more preferably has an area exceeding 2 mm².

### 1-2. Arrangement example 1

Fig. 2 illustrates an arrangement example of the plurality of light receiving elements 12 in the present embodiment.

In the arrangement example of Fig. 2, a plurality of first light receiving elements 121 and one second light receiving element 122 are provided so as to surround one light emitting element 11. As described above, a biological reaction when stress is applied includes two physiological phenomena, **i.e., a** physiological phenomenon derived from a blood vessel and a physiological phenomenon derived from the heart. By using both the physiological phenomena, it is possible to acquire the presence or absence of stress or tension with high accuracy. Therefore, by using the first light receiving elements 121 and the second light receiving element 122 as described above, it is possible to accurately calculate an index of the stress or tension.

In this case, in order to equalize an amount of light received by each light receiving element 12 and facilitate adjustment of an amplification factor in an amplifier, it is preferable that distances from the light emitting element 11 to each light receiving element 12 be substantially the same.

### 1-3. Arrangement example 2

Fig. 3 illustrates an arrangement example of the light receiving elements 12 different from that in Fig. 2.

In the arrangement example of Fig. 3, one first light receiving element 121 and one second light receiving element 122 are arranged adjacent to one light emitting element 11. In the present technology, as described above, one first light receiving element 121 and one second light receiving element 122 may be provided. Even in a case where the number of the first light receiving elements 121 is reduced, biological information can be measured with high accuracy as long as a correlation with the second light receiving element 122 can be established.

### 1-4. Processing unit 100

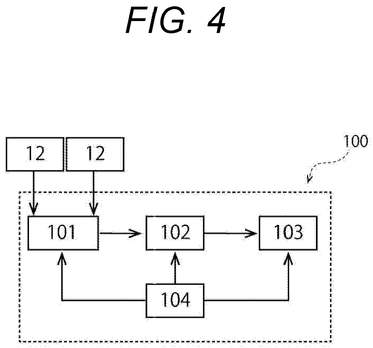

Fig. 4 is a block diagram of a configuration example of the processing unit 100.

The processing unit 100 analyzes biological information (e.g. a state of the autonomic nerve serving as a stress or tension index) on the basis of output from the light receiving elements 12.

The processing unit 100 can include, for example, an extraction unit 101, a recording unit 102, and the analysis unit 103. Further, the processing unit 100 may include the control unit 104 as necessary.

The processing unit 100 may be directly connected to the plurality of light receiving elements 12 or may be connected to the plurality of light receiving elements 12 in a wired or wireless manner.

The extraction unit 101 extracts information from the output of the light receiving elements 12. The extraction unit 101 is achieved by, for example, a low-pass filter.

The low-pass filter is connected to an output end of each light receiving element 12, cuts a high-frequency component (noise component) of a signal output from the light receiving element 12, and outputs the signal in which the high-frequency component has been cut. The signal that has passed through the low-pass filter is transmitted to the recording unit 102 and the analysis unit 103 described later. Note that the low-pass filter may be provided for each light receiving element 12.

The recording unit 102 records the information extracted by the extraction unit 101. The recording unit 102 is achieved by, for example, a central processing unit (CPU), memories such as a read only memory (ROM), a random access memory (RAM), and a flash memory, a hard disk, and the like. The recording unit 102 is connected to an output end of the low-pass filter and holds or updates the signal that has passed through the low-pass filter.

The analysis unit 103 analyzes the signal obtained from each light receiving element 12 and analyzes biological information on the basis of the analysis result. The analysis unit 103 and the control unit 104 are achieved by, for example, a central processing unit (CPU), a field programmable gate array (FPGA), or the like.

Specifically, the analysis unit 103 acquires, for example, a correlation between differences of pieces of information of the respective light receiving elements 12 and determines a state of a living body on the basis of the acquisition result. Examples of the "correlation" here include features such as variance, standard deviation, difference, skewness, kurtosis, correlation, quantile, average value, and median value.

The analysis unit 103 includes, for example, a subtraction unit, a correlation acquisition unit, and a state determination unit.

The subtraction unit is connected to the output end of the low-pass filter and the recording unit 102. The subtraction unit subtracts the signal (old signal) recorded (held or updated) in the recording unit 102 from a signal (new signal) transmitted through the low-pass filter after a predetermined time from the time of recording and outputs a subtraction result (difference). Note that, in a case where the biological information measurement device 10 according to the present technology includes the plurality of light receiving units 12, the subtraction unit may be provided for each low-pass filter provided in each light receiving unit 12.

The correlation acquisition unit is connected to an output end of the subtraction unit. The correlation acquisition unit acquires a correlation between subtraction results (differences) in the subtraction unit (calculates the above features) and outputs the acquisition result (calculation result).

The state determination unit is connected to an output end of the correlation acquisition unit. The state determination unit determines a state of the living body on the basis of the correlation acquired by the correlation acquisition unit.

Note that the present technology may adopt a method of providing a division unit to acquire a division result, instead of the subtraction unit.

Alternatively, the correlation may be acquired from signals recorded in the recording unit (the above features may be calculated) without using subtraction or division, and the acquisition result (calculation result) may be output. In this case, the correlation includes a correlation coefficient and the like.

In the present embodiment, the analysis unit 103 can analyze the biological information by referring to a signal obtained from the second light receiving element 122. Because the second light receiving element 122 has an area exceeding 1 mm², the second light receiving element simultaneously measures information derived from a plurality of blood vessels and thus obtains an averaged signal. Therefore, the second light receiving element has a feature that the signal is hardly affected by a difference in an amount of constriction caused by stress of individual blood vessels. Accordingly, by referring to the signal obtained from the second light receiving element 122, it is possible to accurately calculate information corresponding to the heart rate and the blood pressure, without the difference in the constriction of the individual blood vessels becoming noise.

Further, in this case, the analysis unit 103 can analyze the biological information by referring to the signals obtained from the respective plurality of light receiving elements with each other.

Specifically, it is possible to calculate a shift from a reference of each first light receiving element 121 by referring to the signal of the second light receiving element 122. For example, in a case where the correlation coefficient between the second light receiving element 122 and the first light receiving elements 121 for the past 30 seconds is high, the living body is determined as being in a resting state, whereas, in a case where the correlation coefficient is low, the living body can be determined as being in a stress-applied state. In a case where a plurality of first light receiving elements 121 is arranged, the correlation coefficient may easily decrease due to an influence of, for example, a slight positional shift. However, by using the stable second light receiving element 122 on one side, stable measurement can be performed with a smaller number of light receiving elements.

In the present embodiment, the analysis unit 103 can analyze information derived from a blood vessel on the basis of a signal obtained from the first light receiving element 121. The area of the first light receiving element 121 is 1 mm² or less, which is substantially the same size as the blood vessel, and thus is suitable for calculating, for example, a stress index derived from the blood vessel.

Further, the analysis unit 103 can analyze information derived from the heart on the basis of a signal obtained from the second light receiving element 122. Because the area of the second light receiving element 122 exceeds 1 mm² and signals of a plurality of blood vessels are averaged, the second light receiving element is suitable for observing a reaction derived from the heart, such as, for example, an increase in heart rate or an increase in blood pressure.

Furthermore, when body motion occurs, the analysis unit 103 can ignore a signal from the first light receiving element 121. Because the first light receiving element 121 has substantially the same size as a blood vessel, a measurement value greatly changes only when an installation position is slightly shifted and thus cannot be considered as a continuous measurement value. Therefore, the first light receiving element 121 has a feature of being vulnerable to body motion. Meanwhile, the second light receiving element 122 averages the signals of the plurality of blood vessels and thus can be continuously used even when some body motion occurs. Therefore, in a case where body motion occurs, it is possible to reduce power and a load of calculation processing by stopping the use of the first light receiving element 121.

The control unit 104 controls driving of each of the plurality of light receiving elements 12. Further, the control unit can integrally control the extraction unit 101, the recording unit 102, and the analysis unit 103 described above.

Specifically, the control unit 104 controls driving of the light receiving elements 12 on the basis of signals obtained from the light receiving elements 12. For example, when the above body motion occurs, it is possible to detect occurrence of the body motion on the basis of the plurality of light receiving elements 12 and stop driving of the first light receiving element 121. This makes it possible to reduce power and the load of calculation processing. Further, in a case where it is desired to calculate a stress value with high accuracy, it is possible to drive only the first light receiving element 121. Furthermore, for example, only the second light receiving element may be used to reduce an amount of light emission of the light source for long battery life.

### 2. Biological information measurement system according to present technology

The biological information measurement system according to the present technology includes the above biological information measurement device 10 and an analysis device that analyzes biological information on the basis of signals obtained from the light receiving elements.

Because the biological information measurement device 10 is similar to that described above, the description thereof is omitted here.

Further, the analysis device may have a function similar to that of the analysis unit 103 described above, for example. Because the analysis unit 103 is similar to that described above, the description thereof is omitted here.

In the present technology, the biological information measurement device 10 and/or the analysis device can also be provided in a cloud environment and be connected to each other via a network. In this case, various types of information analyzed by the analysis device on the cloud can also be shared among a plurality of users.

### REFERENCE SIGNS LIST

- 10: Biological information measurement device
- 11: Light emitting element
- 12: Light receiving element
- 121: First light receiving element
- 122: Second light receiving element
- 100: Processing unit
- 101: Extraction unit
- 102: Recording unit
- 103: Analysis unit
- 104: Control unit

## Claims

1. A biological information measurement device comprising:
a light emitting element (11) configured to irradiate a living body with light; and
a first (121) and a second (122) light receiving element configured to receive light emitted from the light emitting element which is scattered in the living body, wherein
the first light receiving element (121) has an area of 0.1mm² or less and the second light receiving element (122) has an area exceeding 1mm²;
further comprising an analysis unit that analyzes biological information on a basis of signals obtained from the first and second light receiving elements;
wherein the analysis unit is configured to analyze information derived from a blood vessel on a basis of a signal obtained from the first light receiving element; and
wherein the analysis unit is configured to analyze information derived from a heart on a basis of a signal obtained from the second light receiving element.

2. The biological information measurement device according to claim 1, comprising a plurality of the first light receiving elements.

3. The biological information measurement device according to claim 1, wherein distances from the light emitting element to the first and second light receiving elements are substantially the same.

4. The biological information measurement device according to claim 1, wherein the analysis unit is configured to analyze the biological information by referring to a signal obtained from the second light receiving element.

5. The biological information measurement device according to claim 4, wherein the analysis unit is configured to analyze the biological information by referring to the signals obtained from the respective first and second light receiving elements with each other.

6. The biological information measurement device according to claim 1, further comprising a control unit configured to control driving of each of the first and second light receiving elements.

7. A biological information measurement system comprising:
a biological information measurement device according to claim 1; and
an analysis device configured to analyze biological information on a basis of signals obtained from the light receiving elements.

8. The biological information measurement system according to claim 7, wherein
the biological information measurement device and
the analysis device
are connected via a network.

## Patentansprüche

1. Vorrichtung zum Messen biologischer Informationen, umfassend:
ein Lichtemissionselement (11), das konfiguriert ist, um einen lebenden Körper mit Licht zu bestrahlen; und
ein erstes (121) und ein zweites (122) Lichtempfangselement, die konfiguriert sind, um von dem Lichtemissionselement emittiertes Licht zu empfangen, das in dem lebenden Körper gestreut wird, wobei
das erste Lichtempfangselement (121) eine Fläche von 0,1 mm² oder weniger aufweist und das zweite Lichtempfangselement (122) eine Fläche aufweist, die 1 mm² übersteigt;
ferner umfassend eine Analyseeinheit, die biologische Informationen auf der Grundlage von Signalen analysiert, die von dem ersten und dem zweiten Lichtempfangselement erhalten werden;
wobei die Analyseeinheit konfiguriert ist, um aus einem Blutgefäß gewonnene Informationen auf der Grundlage eines von dem ersten Lichtempfangselement erhaltenen Signals zu analysieren; und
wobei die Analyseeinheit konfiguriert ist, um von einem Herzen gewonnene Informationen auf der Grundlage eines von dem zweiten Lichtempfangselement erhaltenen Signals zu analysieren.

2. Vorrichtung zum Messen biologischer Informationen nach Anspruch 1, umfassend eine Vielzahl von den ersten Lichtempfangselementen.

3. Vorrichtung zum Messen biologischer Informationen nach Anspruch 1, wobei Abstände von dem Lichtemissionselement zu dem ersten und dem zweiten Lichtempfangselement im Wesentlichen dieselben sind.

4. Vorrichtung zum Messen biologischer Informationen nach Anspruch 1, wobei die Analyseeinheit konfiguriert ist, um die biologischen Informationen durch Bezugnahme auf ein von dem zweiten Lichtempfangselement erhaltenen Signal zu analysieren.

5. Vorrichtung zum Messen biologischer Informationen nach Anspruch 4, wobei die Analyseeinheit konfiguriert ist, um die biologischen Informationen durch Bezugnahme auf die von dem jeweiligen ersten und dem zweiten Lichtempfangselement erhaltenen Signale miteinander zu analysieren.

6. Vorrichtung zum Messen biologischer Informationen nach Anspruch 1, ferner umfassend eine Steuereinheit, die konfiguriert ist, um ein Ansteuern von jedem des ersten und des zweiten Lichtempfangselements zu steuern.

7. System zum Messen biologischer Informationen, umfassend:
eine Vorrichtung zum Messen biologischer Informationen nach Anspruch 1; und
eine Analysevorrichtung, die konfiguriert ist, um biologische Informationen auf der Grundlage von Signalen zu analysieren, die von den Lichtempfangselementen erhalten werden.

8. System zum Messen biologischer Informationen nach Anspruch 7, wobei
die Vorrichtung zum Messen biologischer Informationen und
die Analysevorrichtung
über ein Netzwerk verbunden sind.

## Revendications

1. Dispositif de mesure d'informations biologiques, comprenant :
une unité d'émission de lumière (11) configurée pour irradier un corps vivant avec de la lumière ; et
un premier (121) et un second (122) éléments récepteurs de lumière configurés pour recevoir la lumière émise par l'élément émetteur de lumière qui est diffusée dans le corps vivant, dans lequel
le premier élément récepteur de lumière (121) a une surface égale ou inférieure à 0.1 mm² et le second élément récepteur de lumière (122) a une surface supérieure à 1 mm² ;
comprenant en outre une unité d'analyse qui analyse les informations biologiques sur la base des signaux obtenus à partir des premier et second éléments récepteurs de lumière ;
dans lequel l'unité d'analyse est configurée pour analyser les informations provenant d'un vaisseau sanguin sur la base d'un signal obtenu à partir du premier élément récepteur de lumière ; et
dans lequel l'unité d'analyse est configurée pour analyser les informations provenant d'un coeur sur la base d'un signal obtenu à partir du second élément récepteur de lumière.

2. Dispositif de mesure d'informations biologiques selon la revendication 1, comprenant une pluralité d'éléments émetteurs de lumière.

3. Dispositif de mesure d'informations biologiques selon la revendication 1, dans lequel les distances entre l'élément émetteur de lumière et les premier et second éléments récepteurs de lumière sont sensiblement les mêmes.

4. Dispositif de mesure d'informations biologiques selon la revendication 1, dans lequel l'unité d'analyse est configurée pour analyser les informations biologiques en se référant à un signal obtenu à partir du second élément récepteur de lumière.

5. Dispositif de mesure d'informations biologiques selon la revendication 4, dans lequel l'unité d'analyse est configurée pour analyser les informations biologiques en se référant aux signaux obtenus à partir des premier et second éléments récepteurs de lumière respectifs l'un par rapport à l'autre.

6. Dispositif de mesure d'informations biologiques selon la revendication 1 comprenant en outre une unité de commande configurée pour commander l'entraînement de chacun des premier et second éléments récepteurs de lumière.

7. Système de mesure d'informations biologiques, comprenant :
un dispositif de mesure d'informations biologiques selon la revendication 1 ; et
un dispositif d'analyse configuré pour analyser les informations biologiques sur la base des signaux obtenus à partir des éléments récepteurs de lumière.

8. Système de mesure d'informations biologique selon la revendication 7, dans lequel
le dispositif de mesure d'informations biologiques et
le dispositif d'analyse
sont reliés par l'intermédiaire d'un réseau.
